# EUROPÄISCHE PATENTSCHRIFT

(11) **EP 1 762 270 B1**
(45) Veröffentlichungstag und Bekanntmachung des Hinweises auf die Patenterteilung: **20.08.2008**
(21) Anmeldenummer: 06076542.7
(22) Anmeldetag: 07.08.2006
(51) Int. Cl.: A61N 1/365, A61B 5/053

(54) **Vorrichtung zur Bestimmung von Herzfunktionsparametern**
Device for determining cardiac parameters
Dispositif pour la détermination des paramètres cardiaques

(30) Priorität: 08.09.2005 DE 102005042923
(43) Veröffentlichungstag der Anmeldung: 14.03.2007
(73) Patentinhaber: BIOTRONIK CRM Patent AG, 6341 Baar (CH)
(72) Erfinder: Lippert, Michael, 91522 Ansbach (DE); Czygan, Gerald, 91054 Buckenhof (DE); Paule, Stefan, 96117 Drosendorf (DE)
(74) Vertreter: Lindner-Vogt, Karin L.

(56) Entgegenhaltungen:
- EP-A- 1 384 433
- EP-A- 1 510 173
- EP-A1- 1 348 463
- WO-A-98/19737
- US-A1- 2003 163 058

## Beschreibung

Die Erfindung betrifft eine Vorrichtung zum Bestimmen von Herzfunktionsparametern durch Auswerten des intrakardialen Impedanzverlaufes. Die Vorrichtung weist hierzu eine Impedanzmesseinheit auf, die elektrische Anschlüsse aufweist, an die Elektroden zum Abgeben eines Stromes und zum Erfassen einer Spannung anzuschließen oder permanent angeschlossen sind. Außerdem weist die Vorrichtung eine Auswerteeinheit auf, die mit der Impedanzmesseinheit verbunden und derart ausgebildet ist, dass sie aus einem mittels der Impedanzmesseinheit ermittelten zeitlichen Verlauf der Impedanz einen Herzfunktionsparameter ableiten kann.

Eine geeignete Impedanzmessanordnung ist beispielsweise aus der DE 103 61 143 bekannt. Die dort beschriebene Impedanzmessanordnung ist ausgebildet, aus der intrakardial erfassten Impedanz einen dem Schlagvolumen des Herzens proportionalen Herzfunktionsparameter zu ermitteln, nämlich die Schlagimpedanz. Das Schlagvolumen beschreibt das Blutvolumen, welches vom Herzen während einer Ausstoßphase (Systole) gefördert wird. Zum Ermitteln der Schlagimpedanz wird die Differenz zwischen der enddiastolischen Impedanz (am Ende der Füllungsphase (Diastole) des Herzens) und der endsystolischen Impedanz am Ende der Auswurfphase (Systole) ermittelt. Da die intrakardial erfasste Impedanz maßgeblich von der guten Leitfähigkeit der Blutfüllung des Herzens bestimmt wird, ist die Impedanz bei maximal gefüllten Herzen am Ende der Diastole kleiner als bei maximal kontrahierter Herzkammer am Ende der Systole. Die enddiastolische Impedanz fällt mehr oder weniger mit dem Minimum des Impedanzverlaufes während eines Herzzyklusses zusammen, während die endsystolische Impedanz das Maximum des Impedanzverlaufes während eines Herzzyklusses ist.

Eine auch für die vorliegende Erfindung bevorzugte Impedanzmessanordnung ist eine quadropolare Anordnung wie sie in Figur 1a der DE 103 61 143 abgebildet und in der zugehörigen Beschreibung beschrieben ist. Bei einer derartigen bevorzugten Impedanzmessanordnung wird ein gepulster Konstantstrom zwischen dem Anschluss für eine rechtsventrikuläre Tip-Elektrode und dem Anschluss für eine rechtsventrikuläre Ringelektrode abgegeben. Eine Messung der durch den Strom hervorgerufenen Spannung erfolgt in diesem bevorzugten Fall zwischen dem Anschluss für eine linksventrikuläre Tip-Elektrode und dem Anschluss für eine linksventrikuläre Ringelektrode.

Die hier betroffene, erfindungsgemäße Vorrichtung ist vorzugsweise Bestandteil eines Elektrostimulationsgerätes, welches im bevorzugten Fall implantierbar ist. Ein solches implantierbares Elektrostimulationsgerät kann beispielsweise ein implantierbarer Herzschrittmacher oder auch ein implantierbarer Kardioverter/Defibrillator (ICD) oder eine Kombination aus diesen Implantaten sein.

Eine Vorrichtung gemäß dem Oberbegriff des Anspruchs 1 ist aus EP-A-1 348 463 bekannt.

Ausgehend vom bekannten Stand der Technik wird mit der hier beanspruchten Vorrichtung das Ziel verfolgt, eine erweiterte und verbesserte Herzfunktionsanalyse zu ermöglichen, die im Ergebnis erweiterte Diagnose- und Therapiemöglichkeiten eröffnet. Mit der Erfindung soll eine dafür geeignete Vorrichtung geschaffen werden.

Erfindungsgemäß wird dieses Ziel durch die Merkmale des Anspruchs 1 erreicht.

Die Erfindung beruht auf der Erkenntnis, dass dem Verlauf der intrakardialen Impedanz auch wertvolle Informationen zum Verhalten des Herzens während der Diastole zu entnehmen sind. Die erfindungsgemäße Vorrichtung besitzt demgemäß eine Auswerteeinheit, die zur Ermittlung eines Herzfunktionsparameters nicht nur Impedanzwerte zu Beginn der Diastole und am Ende der Diastole auswertet, sondern auch solche Impedanzwerte, die während der Diastole auftreten. Damit ermöglicht die Vorrichtung erstmals das Erfassen diastolischen Herzversagens mittels intrakardialer Impedanzmessung. Das Erfassen diastolischen Herzversagens wird damit erstmals auch mittels einer implantierbaren Vorrichtung möglich, da sich eine Vorrichtung der hier beschriebenen Art leicht in ein Implantat integrieren lässt.

Herzfehler beeinträchtigen die Pumpleistung eines Herzens. In der Regel ist sowohl die systolische als auch die diastolische Funktion des Herzens beeinträchtigt. Bei einigen Patienten ist hauptsächlich der Relaxations-Prozess des Myokards beeinträchtigt. Patienten mit einem diastolischen Herzfehler zeigen klinische Symptome, die den Symptomen entsprechen, die bei systolischen kongestiven Herzversagen (CHF: Congestive Heart Failure) auftreten, obwohl die systolische Funktion des Herzens normal ist oder nur geringfügig beeinträchtigt. Diastolische Herzfehler beziehungsweise diastolisches Herzversagen können durch eine abnormale Relaxation (Entspannung) des linken Ventrikels verursacht sein, oder durch eine erhöhte passive Steifigkeit des Myokards oder durch andere Faktoren. Die gestörte diastolische Funktion beeinträchtigt die Füllung des Ventrikels während der passiven diastolischen Phase. Die passive Füllung des Ventrikels, das heißt die ventrikuläre Füllungsphase aufgrund der Relaxation des Myokards leistet den größten Beitrag zur Gesamtfüllung des Ventrikels. Die atriale Kontraktion trägt nur zu einem Teil des Blutvolumens bei, das in den Ventrikel fließt. Die beeinträchtigte Relaxation des Ventrikel führt zu einem kompensatorischen Ansteigen des Füllungsdruckes, was wiederum zu einem erhöhten linksatrialen Druck und zu einem erhöhten Kapillarverschlussdruck (PCWP) führt. Wenn nur diastolisches Herzversagen vorliegt, wird das end-diastolische Volumen nicht erhöht und die systolische Funktion bleibt normal. Es wird geschätzt, dass ungefähr ein Drittel aller CHF-Patienten an diastolischem Herzversagen leiden.

Gemäß einer bevorzugten Ausführungsvariante der erfindungsgemäßen Vorrichtung ist die Auswerteeinheit dazu ausgebildet, den maximalen Impedanzabfall während der passiven diastolischen Phase zu ermitteln, also das negative Maximum des Gradienten des Impedanzverlaufes. Als Gradient des Impedanzverlaufes wird im Rahmen dieser Anmeldung die erste Ableitung (Steigung) des Impedanzverlaufes nach der Zeit als die erste zeitliche Ableitung des Impedanzverlaufes bezeichnet. Das negative Maximum des Gradienten des Impedanzverlaufes ist ein nützlicher Herzfunktionsparameter, weil er proportional zur frühen linksventrikulären diastolischen Füllrate ist und den Verlauf der Entspannung des ventrikulären Myokards während der Diastole charakterisiert. Das absolute Maximum der (negativen) ersten Ableitung der Impedanz LV dZ/dt_{Min} während der Diastole, also während der Abnahme der intrakardialen Impedanz, korreliert (nach experimentellen Voruntersuchungen) mit dem entsprechenden Gradienten des Druckverlaufs LV dp/dt_{Min} und ebenso mit der Zeitkonstante des Druckabfalls.

Unter der Annahme, dass die Öffnungsfläche der Mitralklappe konstant ist, ist die negative Steigung (Gradient) des Impedanzverlaufes proportional zur Fließgeschwindigkeit des in den Ventrikel einströmenden Blutes. Daher kann auch die Druckdifferenz zwischen dem linken Ventrikel und dem linken Atrium auf Basis der Bernoullibeziehung, der gemäß die Druckdifferenz zum Quadrat der Fließgeschwindigkeit proportional ist, aus der (negativen) Steigung des Impedanzverlaufes abgeleitet werden. Das Quadrat des Maximums dieser negativen Steigung ist somit proportional zur maximalen Druckdifferenz zwischen dem linken Ventrikel und dem linken Atrium.

Erfindungsgemäß ist die Auswerteeinheit dazu ausgebildet, durch Auswerten des Impedanzverlaufes die Zeitdauer zwischen dem Beginn der Impedanzabnahme und dem darauffolgenden Auftreten des maximalen negativen Gradienten als Wert eines alternativen Herzfunktionsparameters zu bestimmen. Dieser Herzfunktionsparameter wird im Folgenden als Füllbeschleunigungszeit bezeichnet. Der Beginn der Diastole, dass heißt der Beginn des Blutflusses durch die Mitralklappe wird somit durch Erfassen des Beginns der Impedanzabnahme bestimmt. Alternativ kann auch der Zeitpunkt des Auftretens des Impedanzmaximums innerhalb eines Herzzyklusses bestimmt werden.

Die so bestimmte Füllbeschleunigungszeit ist die Zeitdauer zwischen dem Einsetzen des Blutflusses durch die Mitralklappe bis zum Erreichen der maximalen Flussgeschwindigkeit. Diese Füllbeschleunigungszeit korreliert mit der isovolumetrischen Relaxations-Periode und ist ein Maß für die Relaxationsfähigkeit des Myokards. Bei Patienten mit diastolischem Herzversagen ist die Füllbeschleunigungszeit verlängert.

In einer ebenfalls vorteilhaften Variante ist die Auswerteeinheit ausgebildet, durch Auswerten des Impedanzverlaufes eine Füllverzögerungszeit als Herzfunktionsparameter zu bestimmen. Dazu bestimmt die Auswerteeinheit die Zeitdauer zwischen dem Auftreten des maximalen negativen Gradienten des Impedanzverlaufes und dem Apex einer parabolischen Näherungsfunktion des Impedanzverlaufes beginnend mit dem Auftreten des maximalen negativen Gradienten des Impedanzverlaufes. Statt das Ende der Füllverzögerungszeit mit Hilfe einer parabolischen Näherungsfunktion zu bestimmen kann der Impedanzverlauf vom Zeitpunkt des Auftretens des maximalen negativen Gradienten an auch durch eine Exponentialfunktion angenähert werden, deren Zeitkonstante LVτ(Z) dann die Füll-Verzögerungszeit wiedergibt.

In einer weiteren vorteilhaften Variante ist die Auswerteeinheit dazu ausgebildet, durch Auswerten des Impedanzverlaufes das Verhältnis des negativen Maximums des Gradienten des Impedanzverlaufes zwischen dem Beginn der Abnahme der Impedanz und einer darauffolgenden atrialen Kontraktion zum negativen Maximum des Gradienten des Impedanzverlaufes zwischen dieser atrialen Kontraktion und dem darauffolgenden Wiederanstieg des Impedanzverlaufes zu bestimmen. Auf diese Weise wird das Verhältnis der maximalen Fließgeschwindigkeit der E-Welle, die den ventrikulären Beitrag zur Füllung des linken Ventrikels beschreibt, und der A-Welle, die den atrialen Beitrag zur Füllung des linken Ventrikels beschreibt, bestimmt. Das so bestimmte Verhältnis wird im Folgenden auch als V_{E}/V_{A}-Verhältnis bezeichnet.

Schließlich ist die Bestimmung der Dauer der Diastole ein weiterer Herzfunktionsparameter, zu dessen Bestimmung die Auswerteeinheit vorzugsweise ausgebildet ist. Diese Zeitdauer bestimmt die Auswerteeinheit in der bevorzugten Ausführungsform dadurch, das die Auswerteeinheit zunächst den Beginn des Abfalls des Impedanzverlaufes nach Erreichen des Impedanzmaximums während eines Herzzyklusses erfasst und dann den Zeitpunkt des Auftretens des nachfolgenden Impedanzminimums.

Neben den zuvor genannten Varianten der Vorrichtung mit einer Auswerteeinheit, die zum Bestimmen der genannten Werte von Herzfunktionsparametern ausgebildet ist, die die Diastole kennzeichnet, kann die Auswerteeinheit in einer bevorzugten Ausführungsvariante ausgebildet sein, zusätzlich Werte solcher Herzfunktionsparameter zu bestimmen, die das Verhalten des Herzens während einer Systole beschreiben. Dazu ist die Auswerteeinheit ausgebildet, den Verlauf der intrakardialen Impedanz während der Systole, also der Auswurfphase der Herzkammer, die mit einer Kontraktion des jeweiligen Ventrikels einhergeht, zu bestimmen.

Ein solcher systolischer Herzfunktionsparameter, zu dessen Bestimmung die Auswerteeinheit vorzugsweise ausgebildet ist, ist das positive Maximum des Gradienten des Impedanzverlaufes während der Systole. Während der Systole steigt die Impedanz an, weil Blut aus dem Ventrikel verdrängt wird. Die Steigung des Impedanzverlaufes, also die erste zeitliche Ableitung des Impedanzverlaufes während der Auswurfphase korrespondiert mit der Änderungsrate des linksventrikulären Volumens. Unter der Annahme, dass die Aortenklappe während der Auswurfphase einen konstanten Querschnitt hat, ist die Steigung der Impedanz proportional zur Ausströmgeschwindigkeit des Blutes. Dies erlaubt es, die Druckdifferenz zwischen dem linken Ventrikel und der Aorta unter Berücksichtigung der Bernoullibeziehung (Δp ~ v²) aus der Steigung der Impedanz zu berechnen. Das Quadrat des Steigungsmaximums ist somit proportional zum Maximum der Druckdifferenz zwischen linken Ventrikel und Aorta.

In einer weiteren bevorzugten Variante ist die Auswerteeinheit dazu ausgebildet, dass Maximum der Steigung des Gradienten des Impedanzverlaufes, also dessen zweite Ableitung nach der Zeit zu bilden. Unter Berücksichtigung der Windkessel-Funktion der Aorta ist die zweite zeitliche Ableitung des Impedanzverlaufes LVd²Z/dt² , multipliziert mit der ersten Ableitung LVdZ/dt , ein Marker für die maximale linksventrikuläre Druckänderung LVdp/dt_{Max}, die ein Maß für die Kontraktilität des linken Ventrikels ist. Als Windkesselfunktion der Aorta wird die Eigenschaft des herznahen Aortaabschnittes bezeichnet, sich elastisch ausdehnen und wieder kontrahieren zu können, und auf diese Weise das Pulsieren des Blutflusses etwas zu glätten.

Gemäß einer weiteren bevorzugten Variante der Auswerteeinheit ist diese ausgebildet durch Auswerten des Impedanzverlaufes die Zeitdauer der Vor-Auswurf-Phase (Präejektionsphase, Pre-Ejection-Period, PEP) als Herzfunktionsparameter zu bestimmen, indem die Auswerteeinheit die Zeitdauer zwischen der elektrischen Aktivierung des ventrikulären Myokards und dem Beginn des Impedanzanstieges bestimmt. Den Zeitpunkt der elektrischen Aktivierung des ventrikulären Myokards kann die Auswerteeinheit durch Erfassen einer R-Zacke im vorzugsweise intrakardial aufgenommenen Elektrokardiogramm (EKG) oder durch Erfassen des Zeitpunktes einer Abgabe eines linksventrikulären Stimulationsimpulses bestimmen. Um den Beginn des Impedanzanstieges zu bestimmen, kann die Auswerteeinheit dazu ausgebildet sein, den Zeitpunkt zu bestimmen, zu dem die intrakardiale Impedanz einen auf das Impedanzminimum bezogenen Schwellwert überschreitet.

Ebenfalls bevorzugt ist eine Ausführungsvariante, bei der die Auswerteeinheit dazu ausgebildet ist, durch Auswerten des Impedanzverlaufes die Zeitdauer der linksventrikulären Auswurfzeit zu bestimmen, indem die Auswerteeinheit die Zeitdauer zwischen dem Beginn des Impedanzanstieges und dem Maximum des Impedanzverlaufes bestimmt. Der Beginn des Impedanzanstieges, also der Zeitpunkt, von dem an die intrakardiale Impedanz im Verlaufe der Zeit wieder zunimmt, korrespondiert mit dem Öffnen der Aortenklappe. Das Maximum des Impedanzverlaufes entspricht dem Ende der Auswurfphase und korrespondiert mit dem Schließen der Aortenklappe.

Auch ist eine Ausführungsvariante der Auswerteeinheit bevorzugt, bei der die Auswerteeinheit dazu ausgebildet ist, durch Auswerten des Impedanzverlaufes die linksventrikuläre Auswurfbeschleunigungszeit als Herzfunktionsparameter zu bestimmen. Dazu ist die Auswerteeinheit ausgebildet, die Zeitdauer zwischen dem Beginn des Impedanzanstieges und dem darauf folgenden Auftreten des maximalen positiven Gradienten des Impedanzverlaufes zu bestimmen. Die so bestimmte Beschleunigungszeit ist die Zeit zwischen dem Beginn der Auswurfphase und dem Auftreten der maximalen Fließgeschwindigkeit des Blutes.

In ähnlicher Weise kann die Auswerteeinheit in einer bevorzugten Variante dazu ausgebildet sein, durch Auswerten des Impedanzverlaufes die linksventrikuläre Auswurfverzögerungszeit zu bestimmen. Dazu bestimmt die Auswerteeinheit die Zeitdauer zwischen dem Auftreten des maximalen positiven Gradienten des Impedanzverlaufes während der Systole und dem darauf folgenden Maximum des Impedanzverlaufes.

Das Verhältnis zwischen der Auswurfbeschleunigungszeit und der Auswurfverzögerungszeit wird durch eine mögliche Stenose der Aorta beeinflusst: Eine zunehmende Stenose (Verengung) der Aorta vergrößert den Anteil der Auswurfbeschleunigungszeit an der Gesamtdauer der Systole.

In einer weiteren bevorzugten Ausführungsvariante der Vorrichtung ist die Auswerteeinheit dazu ausgebildet, die elektromechanische Verzögerungszeit als die Zeitdifferenz zwischen der elektrischen Aktivierung des Ventrikels und dem Einsetzen des Impedanzanstieges während der systolischen Phase zu bestimmen. Dazu ist die Auswerteeinheit mit einer Impedanzmesseinheit verbunden, die derart mit Impedanzmesselektroden zusammenwirkt, dass sie ein lokales rechtsventrikuläres oder linksventrikuläres Impedanzsignal erfasst, beispielsweise mittels einer unipolaren oder bipolaren Impedanzmessanordnung im rechten oder linken Ventrikel. Dadurch, dass die für die Bestimmung der elektromechanischen Verzögerungszeit herangezogene Impedanz die lokale rechtsventrikuläre oder linksventrikuläre Impedanz ist, unterscheidet sich die elektromechanische Verzögerungszeit von der Dauer der Vor-Auswurfperiode (Pre-Ejection Period, PEP), die auf Basis eines über das ganze Herz gemessenen Impedanzverlaufes bestimmt wird. Der Zeitpunkt der elektrischen Aktivierung des jeweiligen Ventrikels kann die Auswerteeinheit durch Auswerten eines intrakardialen Elektrokardiogramms bestimmen, indem die Auswerteeinheit den Zeitpunkt des Auftretens einer R-Zacke in diesem Elektrokardiogramm bestimmt.

Vorzugsweise besitzt die Vorrichtung einen Speicher, um die zuvor beschriebenen, durch Auswerten des intrakardialen Impedanzverlaufes bestimmten Herzfunktionsparameter für diagnostische Zwecke aufzeichnen zu können.

Der Speicher weist vorzugsweise wenigstens zwei Speicherbereiche auf, von denen ein erster Speicherbereich für Herzfunktionsparameterwerte vorgesehen ist, die während einer Phase physischer Aktivität des Patienten erfasst wurden, und ein zweiter Speicherbereich für das Speichern von Herzfunktionsparameterwerten dient, die während einer Ruhephase des Patienten erfasst wurden. Um Ruhephasen des Patienten von Aktivitätsphasen unterscheiden zu können, weist die Vorrichtung vorzugsweise eine Aktivitätserfassungseinheit auf. Diese kann beispielsweise mit einem physiologischen Sensor zum Bestimmen des hämodynamischen Bedarfes des Patienten gekoppelt sein, wie er in ratenadaptiven Herzschrittmachern häufig zum Zwecke der Adaption der Stimulationsrate eingesetzt wird. Ein solcher physiologischer Sensor kann beispielsweise ein Beschleunigungssensor sein. Die Auswerteeinheit ist direkt oder indirekt mit der Aktivitätserfassungseinheit verbunden und dazu ausgebildet, die von der Auswerteeinheit bestimmten Herzfunktionsparameterwerte je nach Ausgangssignal der Aktivitätserfassungseinheit in dem ersten oder in dem zweiten Speicherbereich des Speichers zu speichern.

Vorteilhaft ist es, wenn die Auswerteeinheit weiterhin ausgebildet ist, Herzfunktionsparameterwerte für einen jeweiligen Herzfunktionsparameter über die Zeit zu mitteln. Dazu greift die Auswerteeinheit auf in dem Speicher gespeicherte Herzfunktionsparameterwerte zurück.

Die Auswerteeinheit kann weiterhin ausgebildet sein, auf Basis der gespeicherten Herzfunktionsparameterwerte Histogramme zu erstellen. Dazu weist die Vorrichtung vorzugsweise eine Aktivitätserfassungseinheit auf, die dazu ausgebildet ist, mehrere Zustände physischer Aktivität unterschiedlicher Intensität zu differenzieren. Der Speicher weist in dieser Ausführungsvariante mehrere Speicherbereiche auf und die Auswerteeinheit ist ausgebildet, Herzfunktionsparameterwerte in Abhängigkeit des Ausgangssignals der Aktivitätserfassungseinheit je nach Intensität der zugeordneten physischen Aktivität in einem der Speicherbereiche zu speichern.

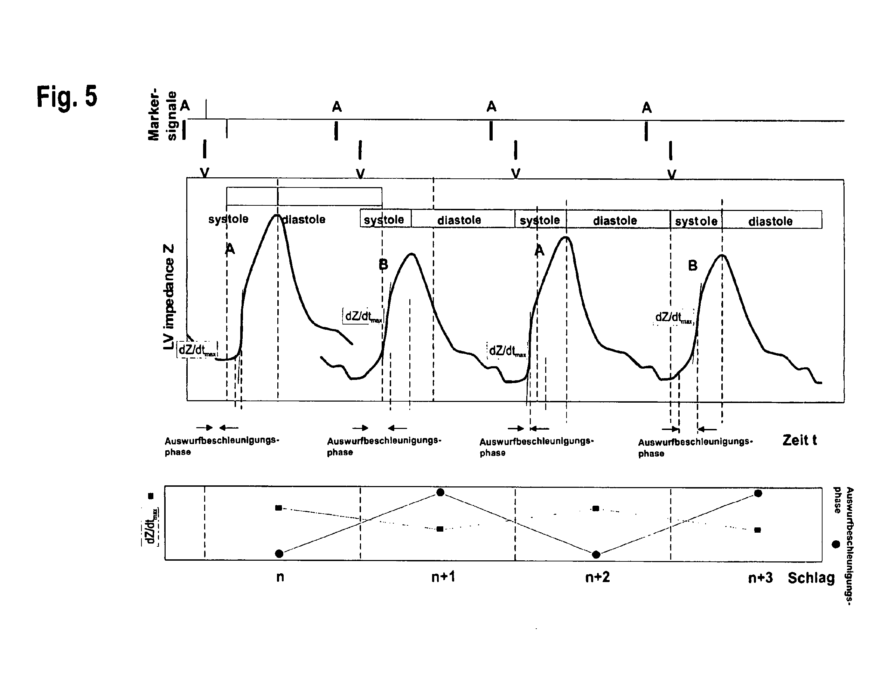

Schließlich kann die Auswerteeinheit zusätzlich ausgebildet sein, Herzfunktionsparameterwerte eines Herzfunktionsparameters daraufhin zu untersuchen, ob der jeweilige Herzfunktionsparameter von Herzzyklus zu Herzzyklus (von Herzschlag zu Herzschlag; Beat to Beat) alterniert. Ein solches alternierendes Verhalten eines Herzfunktionsparameters von Schlag zu Schlag wird im Folgenden auch als Alternanz bezeichnet. Eine nähere Erläuterung der Alternanz erfolgt im Zusammenhang mit Figur 5 in der nachfolgenden detaillierten Beschreibung der Erfindung.

Besonders vorteilhaft ist es, wenn die von der Auswerteeinheit ermittelten und in dem Speicher gespeicherten Herzfunktionsparameterwerte sowie davon abgeleitete Werte wie beispielsweise Mittelwerte über die Zeit oder Histogrammwerte mittels einer Telemetrieeinheit der Vorrichtung zu einem Empfänger außerhalb der Vorrichtung übertragen werden können. Dazu ist vorzugsweise eine Telemetrieeinheit vorgesehen, die mit dem Speicher verbunden ist und wenigstens eine Sendeeinheit zum drahtlosen Übertragen von Daten aufweist. Eine derartige Vorrichtung ermöglicht es, die verschiedenen in dem Speicher gespeicherten Werte zu einer zentralen Datenverarbeitungseinheit zu übertragen, die über eine größere Speicherkapazität und über eine größere Rechenkapazität verfügen kann und daher zu einer weitergehenden Auswertung der von der Auswerteeinheit ermittelten Daten geeignet ist.

Die Erfindung soll nun anhand eines Ausführungsbeispiels mit Bezug auf die Figuren näher erläutert werden. Von den Figuren zeigen:
- Figur 1a:: eine erfindungsgemäße Vorrichtung in Form eines Herzschrittmachers samt daran angeschlossener Elektroden. Die Lage der Elektroden in Bezug auf ein menschliches Herz ist skizzenhaft dargestellt.
- Figur 1b:: eine skizzenhafte Darstellung des zur Impedanzmessung eingespeisten Stroms;
- Figur 2a:: ein Blockschaltbild wesentlicher Komponenten des Herzschrittmachers aus Figur 1;
- Figur 2b:: ein Blockschaltbild wesentlicher Komponenten des Herzschrittmachers aus Figur 1 in einer zu Figur 2a alternativen Ausführungsvariante;
- Figur 3a:: eine skizzenhafte Darstellung eines Elektrokardiogramms über etwas mehr als einen Herzzyklus;
- Figur 3b:: einen typischen Verlauf der intrakardialen Impedanz in synoptischer Darstellung zum Verlauf des Elektrogramms in Figur 3a;
- Figur 4:: eine differenziertere Darstellung des Verlaufs der intrakardialen Impedanz zur Erläuterung wesentlicher, mit dem Impedanzverlauf in Beziehung stehender Parameter; und
- Figuren 5a und b:: den Verlauf der intrakardialen Impedanz über mehrere Zyklen zur Darstellung einer Alternanz.

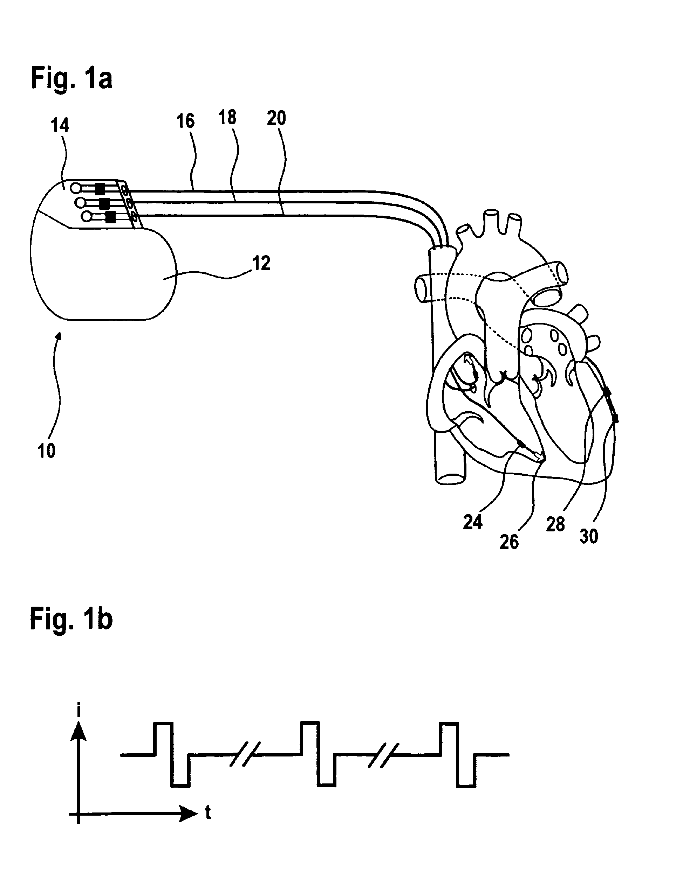

In Figur 1a ist ein Implantat 10 dargestellt, das beispielsweise ein Herzschrittmacher ist. Das Implantat 10 besitzt ein hermetisch dichtes Metallgehäuse 12 und einen Anschlusskopf (Header) 14 aus transparentem Kunststoff, der mehrere Buchsen zum Anschluss von Elektrodenleitungen aufweist. Die Elektrodenleitungsanschlüsse im Header 14 sind elektrisch mit einer Steuerschaltung im Inneren des Gehäuses des Herzschrittmachers 10 verbunden.

An die Elektrodenleitungsanschlüsse sind insgesamt drei Elektrodenleitungen angeschlossen, nämlich eine rechtsatriale Elektrodenleitung 16, eine rechtsventrikuläre Elektrodenleitung 18 und eine linksventrikuläre Elektrodenleitung 20.

Die rechtsatriale Elektrodenleitung 16 trägt eine rechtsatriale Stimulationselektrode 22. Die rechtsventrikuläre Elektrodenleitung 18 trägt eine rechtsventrikuläre Ringelektrode 24 und eine rechtsventrikuläre Spitzenelektrode (Tip-elektrode) 26. Die linksventrikuläre Elektrodenleitung 20 ist über das rechte Atrium des in Figur 1a schematisch dargestellten Herzens und den Coronar Sinus des Herzens bis in die Peripherie des linken Ventrikels geführt. Die linksventrikuläre Elektrodenleitung 20 trägt eine linksventrikuläre Ringelektrode 28 und eine linksventrikuläre Spitzenelektrode 30.

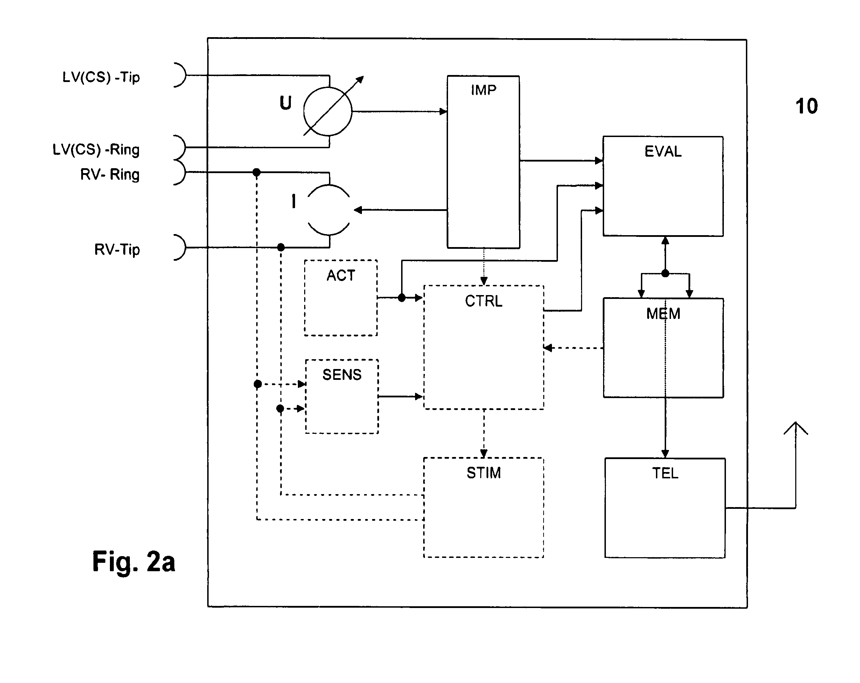

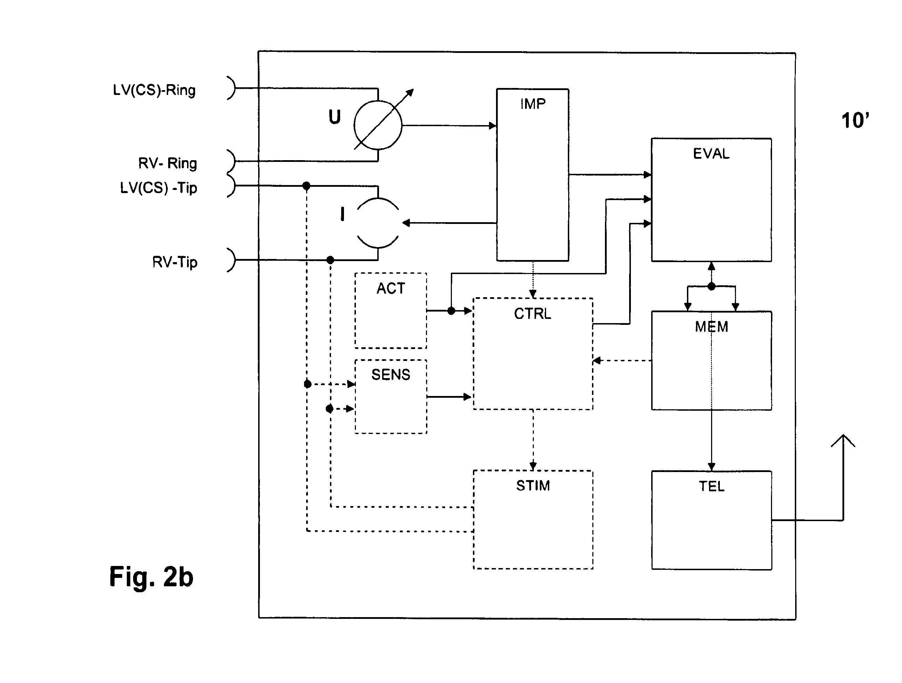

Für die hier interessierende Impedanzmessung besitzt das Implantat 10 eine Impedanzmesseinheit (siehe Figur 2) die der hier dargestellten, bevorzugten Ausführungsvariante (Figur 2a) zum Zwecke der Impedanzmessung mit der rechtsventrikulären Ringelektrode 24 und der rechtsventrikulären Spitzenelektrode 26 sowie der linksventrikulären Ringelektrode 28 und der linksventrikulären Spitzenelektrode 30 verbunden ist.

Die Abgabe eines biphasischen, gepulsten Messstroms, wie er in Figur 1b skizzenhaft dargestellt ist, erfolgt über die rechtsventrikuläre Ringelektrode 24 und die rechtsventrikuläre Spitzenelektrode 26, also im rechten Ventrikel. Die Messung der durch den Strom hervorgerufenen Spannung erfolgt über die linksventrikuläre Ringelektrode 28 und die linksventrikuläre Spitzenelektrode 30.

Wie Figur 1b zu entnehmen ist, wird der Strom zur Impedanzmessung in biphasischen Impulsen abgegeben, wobei zwei gegenphasige Konstantstromimpulse unmittelbar aufeinander folgen und jeweils ein Impulspaket bilden. Die einzelnen Impulspakete haben einen zeitlichen Abstand voneinander, der wesentlich größer ist als die Dauer des jeweiligen Impulspaketes. Die beiden Gleichstromimpulse innerhalb des Impulspaketes besitzen jeweils die gleiche Stärke, jedoch mit unterschiedlicher Polung und sind jeweils gleich lang. Typische Werte für die Stärke der Gleichstromimpulse liegen zwischen 50 µA und 600 µA. Eine typische Impulsdauer eines einzelnen Strompulses ist 15 µs.

Der Abstand der Impulspakete voneinander ist beispielsweise 500 Mal größer als die Dauer eines Stromimpulses. Anders als in Figur 1b dargestellt können die beiden Gleichstromimpulse eines Impulspaketes auch mit einem zeitlichen Abstand aufeinander folgen, der der Dauer eines Gleichstromimpulses entspricht. Zwischen zwei gegenphasigen Gleichstromimpulsen eines Impulspaketes ergibt sich dann jeweils eine Lücke von der Dauer eines Gleichstromimpulses, während der kein Gleichstrom abgegeben wird. Eine weitere Variante besteht darin, dass die Impulspakete phasenalternierend abgegeben werden, das heißt, dass streng abwechselnd ein Impulspaket beispielsweise mit einem negativen Gleichstromimpuls beginnt und mit einem positiven Gleichstromimpuls endet und das darauf folgende Impulspaket mit einem positiven Gleichstromimpuls beginnt und mit einem negativen Gleichstromimpuls endet und so fort. Durch diese phasenalternierende Abgabe von Impulspaketen wird die Belastung des Myokards vermindert und es werden Artefakte vermieden. Figuren 2a und 2b zeigen jeweils ein Blockschaltbild mit den in bezug auf die hier betroffene Erfindung wesentlichen Komponenten einer Schaltung im Inneren des Gehäuses 12 des Herzschrittmachers 10. Diese Bestandteile sind eine Impedanzmesseinheit IMP, die einerseits mit einem Konstantstromgenerator I verbunden ist, der einen gepulsten, biphasischen Konstantstrom erzeugt und über einen Anschluss RV-Ring für die rechtsventrikuläre Ringelektrode 24 und einen Anschluss RV-Tip für rechtsventrikuläre Spitzenelektrode 26 abgibt. Außerdem ist die Impedanzmesseinheit mit einer Spannungsmesseinheit U verbunden, die ihrerseits mit zwei Anschlüssen verbunden ist, über die die jeweilige Spannung erfasst wird, die der zu Impedanzmesszwecken abgegebene Konstantstrom hervorruft. Die Spannungsmesseinheit U ist im Falle der bevorzugten Ausführungsvariante gemäß Figur 2a mit einem Anschluss LV-Ring für die linksventrikuläre Ringelektrode 28 sowie mit einem Anschluss LV-Tip für die linksventrikuläre Spitzenelektrode 30 verbunden. In einer alternativen Ausführungsvariante (siehe Figur 2b) ist die Spannungsmesseinheit U einerseits mit einem Anschluss RV-Tip für die rechtsventrikuläre Spitzenelektrode 26 und andererseits mit einem Anschluss LV-Tip für die linksventrikuläre Spitzenelektrode 30 verbunden. Die Stromeinspeisung erfolgt in dieser Ausführungsvariante über die rechtsventrikuläre Ringelektrode 24 am Anschluss RV-Ring und die linksventrikuläre Ringelektrode 28 am Anschluss LV-Ring.

Die Impedanzmesseinheit IMP ist ausgebildet, den Verlauf der intrakardialen Impedanz in zeitabgetasteter Form (gesampelt) zu erfassen und ein entsprechendes Impedanzverlaufssignal an einer Auswerteeinheit EVAL weiterzugeben. Die Auswerteeinheit EVAL ist ausgebildet, zumindest den Impedanzverlauf während der Diastole gemäß einer der zuvor beschriebenen Varianten auszuwerten.

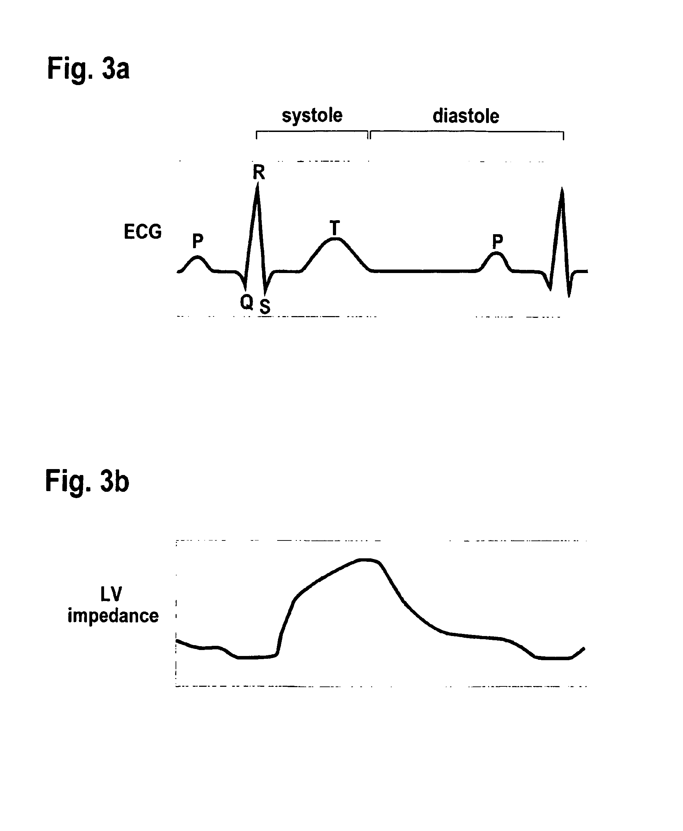

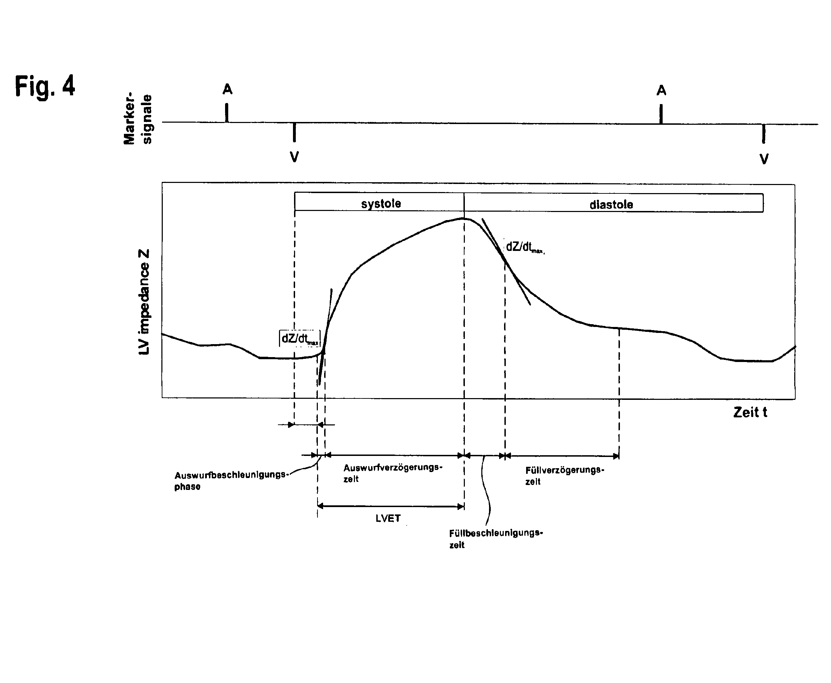

Ein typischer Verlauf der intrakardialen Impedanz ist mit Figur 3b und Figur 4 abgebildet. Eine Erläuterung des Impedanzverlaufes wird im Zusammenhang mit der Beschreibung dieser Figuren erfolgen. Im Zusammenhang mit Figur 4 werden insbesondere diejenigen Zeitpunkte der Steigungen (Gradienten) Maxima und Minima des Impedanzverlaufes erläutert werden, die für die Bestimmung der zuvor genannten Herzfunktionsparameter herangezogen werden.

Die Auswertung des Impedanzverlaufes durch die Auswerteeinheit erfolgt in einigen Fällen sinnvoller Weise mit Bezug auf ein vorzugsweise intrakardial erfasstes Elektrokardiogramm. Dies ist vorstehend insbesondere mit Bezug auf die Bestimmung der Dauer der Vor-Auswurfperiode (Pre-Ejection Period, PEP) bereits erwähnt. Zum Erfassen eines intrakardialen Elektrokardiogramms (iEGM: intracardiac Electrogram) weisen auch bekannte Herzschrittmacher bereits entsprechende Sensingeinheiten SENS auf, die beispielsweise - wie im Ausführungsbeispiel gemäß Figuren 2a und 2b dargestellt - mit dem Anschluss für die rechtsventrikuläre Ringelektrode 24 und die rechtsventrikuläre Spitzenelektrode 28 verbunden ist. Das intrakardial erfasste Elektrokardiogramm gibt die elektrischen Potentiale wieder, die mit der Kontraktion respektive der Expansion des Myokards einhergehen. Ein typischer Elektrokardiogrammverlauf ist in Figur 3a abgebildet. Aus dem Elektrokardiogramm gehen insbesondere die Potentiale hervor, die mit einer Depolarisation des ventrikulären Myokards einhergehen und damit mit einer ventrikulären Kontraktion. Diese Potentiale sind dem Elektrokardiogramm als R-Zacke zu entnehmen. Die Repolarisation des Myokards und damit der Beginn der Entspannung (Relaxation) des Myokards fällt mit der T-Welle im Elektrokardiogramm zusammen. Eine P-Welle kennzeichnet die Kontraktion des Atriums. Üblicher Weise besitzen Herzschrittmacher eine Steuereinheit CTRL die ausgebildet ist, den Zeitpunkt des Auftretens einer R-Zacke und eine P-Welle zu detektieren und ein entsprechendes Markersignal zu erzeugen, das eine ventrikuläre Kontraktion (V) beziehungsweise eine atriale Kontraktion (A) kennzeichnet. Die entsprechenden, den Zeitpunkt des Auftretens einer R-Zacke beziehungsweise einer P-Welle kennzeichnenden Markersignale V und A sind auch in den Figuren 4 und 5 wiedergegeben.

Weiterhin ist den Figuren 2a und 2b zu entnehmen, dass der Herzschrittmacher 10 beziehungsweise 10' einen Aktivitätssensor ACT besitzt, der zum einen in üblicher Manier dazu dient, Phasen körperlicher Aktivität eines Patienten zu erfassen. Körperliche Aktivität geht üblicher Weise mit einem erhöhten metabolischen Bedarf einher, so dass bei bekannten Herzschrittmachern das Erfassen einer erhöhten körperlichen Aktivität des Patienten mit Hilfe des Aktivitätssensors ACT zu Erhöhung der Stimulationsrate führt. Im Zusammenhang mit der vorliegenden Erfindung wird das Ausgangssignal des Aktivitätssensors ACT zusätzlich der Auswerteeinheit EVAL zur Auswertung des Impedanzsignales zugeführt. Die Auswerteeinheit EVAL nutzt das Ausgangssignal des Aktivitätssensors ACT um ermittelte Herzfunktionsparameterwerte in einem der beiden Speicherbereiche des Speichers MEM abzulegen je nachdem, ob ein Herzfunktionsparameterwert während einer Phase physischer Aktivität des Patienten erfasst wurde oder während einer Ruhephase des Patienten.

Der Speicher MEM ist schließlich mit einer Telemetrieeinheit TEL verbunden, die wenigstens eine Sendereinheit enthält und dazu ausgebildet ist, Daten aus dem Speicher MEM drahtlos an einen Empfänger außerhalb des Implantates 10 zu übertragen.

Nun soll anhand von Figur 3 kurz der Zusammenhang zwischen einem Elektrokardiogramm (ECG) und dem Verlauf der linksventrikulären Impedanz erläutert werden. Figur 3 a zeigt ein typisches Elektrokardiogramm und Figur 3b zeigt in synoptischer Zuordnung zum Elektrokardiogramm einen typischen Verlauf der linksventrikulären Impedanz.

Wie erwähnt, ergibt sich das Elektrokardiogramm aus elektrischen Potentialen des Myokards, wie sie zusammen mit der Kontraktion und der Relaxation des Myokards auftreten. Eine Kontraktion des Herzmuskelgewebes - hier die Kontraktion des linken Ventrikels - wird durch ein elektrisches Potential (eine elektrische Erregung) ausgelöst, welches zu einer Depolarisation der Herzmuskelzellen führt und die sich ausgehen von einem Erregungsort auf das gesamte Myokard einer Herzkammer ausbreitet und so zur Kontraktion der Herzkammer führt. Diese, zur Depolarisation der Herzmuskelzellen und damit zur Kontraktion des Herzgewebes führende elektrischen Potentiale sind im Elektrokardiogramm als R-Zacke zu erkennen. Die Repolarisation des Herzgewebes, die mit der Entspannung (Relaxation) des Myokards einhergeht, fällt mit der im Elektrokardiogramm erkennbaren T-Welle zusammen. Eine P-Welle ergibt sich aus den elektrischen Potentialen, die mit der Kontraktion des Atriums einhergehen.

Wie dem Verlauf der linksventrikulären Impedanz in Figur 3b zu entnehmen ist, besitzt die Impedanz etwa zum Zeitpunkt des Auftretens der R-Zacke ein Minimum. Zu diesem Zeitpunkt besitzt der Ventrikel sein größtes Volumen und hat daher die geringste Impedanz. Mit einer geringen Verzögerung nach Auftreten der R-Zacke beginnt sich der Ventrikel zu kontrahieren, so dass die Impedanz steigt, bis beim maximal kontrahierten Ventrikel ihr Maximum erreicht. Die Phase des Anstiegs der linksventrikulären Impedanz geht mit der Auswurfphase (Systole) des Herzens einher, bei der Blut aus dem Ventrikel durch die Aortenklappe hindurch in die Aorta gepresst wird. Nachdem der Ventrikel seinen maximal kontrahierten Zustand erreicht hat, repolarisiert das Herzmuskelgewebe (Myokard). Der Beginn der Repolarisation ist im Elektrokardiogramm als T-Welle zu erkennen und führt dazu, dass die linksventrikuläre Impedanz nach Erreichen des Impedanzmaximums wieder abfällt. Der Abfall der linksventrikulären Impedanz spiegelt das sich mit zunehmender Relaxation des Myokards vergrößernde Volumen des linken Ventrikels wieder. Eine Kontraktion des Atrium, der im Elektrokardiogramm einer P-Welle vorangeht, führt zu einer weiteren Füllung des Ventrikels und einer entsprechenden Volumenvergrößerung und damit zu einem weiteren Impedanzabfall, bis es schließlich zu einer erneuten Kontraktion des Ventrikels kommt.

In Figur 4 sind schließlich die Zeitpunkte und Werte dargestellt, die die Auswerteeinheit zum Bestimmen der Herzfunktionsparameterwerte ermittelt. Auch sind in Figur 4 die meisten der Herzfunktionsparameter eingetragen, deren Werte zu bestimmen die Auswerteeinheit ausgebildet ist.

Darüber hinaus ist in Figur 4 angedeutet, dass die Auswerteeinheit EVAL die Zuordnung von dem Impedanzverlauf zu entnehmenden Werten und Zeitpunkten zu den Zeitpunkten des Auftretens einer R-Zacke und einer P-Welle im Elektrokardiogramm durch Auswerten entsprechender Markersignale V und A vornimmt.

Figur 4 ist zu entnehmen, dass sich die Systole, also die Auswurfphase vom Zeitpunkt der Depolarisierung des ventrikulären Myokards - gekennzeichnet durch einen V-Marker - bis zum Erreichen des minimalen ventrikulären Kammervolumens - gekennzeichnet durch das Maximum des Impedanzverlaufes - erstreckt. Die im Zusammenhang mit der Erfindung besonders interessierende Diastole erstreckt sich in zeitlicher Hinsicht vom Zeitpunkt des Auftretens des Impedanzmaximums bis zur nächsten ventrikulären Depolarisation.

Die Vor-Auswurfsphase PEP erstreckt sich vom Beginn der ventrikulären Depolarisation (V-Marker) bis zum deutlichen Einsetzen eines Impedanzanstiegs. Die Auswurfbeschleunigungsphase (Ejection Acceleration Time) erstreckt sich von diesem deutlichen Anstieg der linksventrikulären Impedanz bis hin zum Erreichen des Zeitpunkts zu dem der Impedanzverlauf seine größte positive Steigerung hat, das heißt der Gradient des Impedanzverlaufes am größten ist. Vom Zeitpunkt des Auftretens der maximalen Steigung (maximaler Gradient) des Impedanzverlaufes bis zum Erreichen des Impedanzmaximums erstreckt sich die Auswurfverzögerungszeit (Ejection Deceleration Time). Auswurfbeschleunigungszeit und Auswurfverzögerungszeit ergeben zusammen genommen die linksventrikuläre Auswurfzeit (LVET). Mit Erreichen der maximalen linksventrikulären Impedanz endet die Auswurfphase des Herzens, also die Systole, und es beginnt die Füllungsphase, also die Diastole.

Die Diastole beginnt mit der Füllbeschleunigungszeit (Filling Acceleration Time), die sich in zeitlicher Hinsicht vom Auftreten des Maximums des Impedanzverlaufes bis zum Zeitpunkt des Auftretens der maximalen negativen Steigung des Impedanzverlaufes (maximaler negativer Gradient dZ/dt_{Min}) erstreckt. Die Füllverzögerungszeit (Filling Deceleration Time) beginnt mit dem Zeitpunkt des Auftretens des maximalen negativen Gradienten und erstreckt sich bis zum Auftreten des Apexes einer parabolischen Näherungsfunktion des Impedanzverlaufes, beginnend mit dem Zeitpunkt des Auftretens der maximalen negativen Steigung.

Mit Bezug auf Figur 5 soll nun die bereits angedeutete Bestimmung der Alternanz eines Herzfunktionsparameters durch die Auswerteeinheit EVAL näher erläutert werden.

Figur 5a zeigt einen typischen, alternierenden Impedanzverlauf über mehrere Zyklen in zeitlichem Bezug zum Auftreten atrialer und ventrikulärer Ereignisse (Depolarisationen) gekennzeichnet durch A-Marker und V-Marker.

Als zweiter Herzfunktionsparameter, der aus dem linksventrikulären Impedanzverlauf abzuleiten ist, ist in Figur 5a die jeweilige Auswurfbeschleunigungszeit (Ejection Acceleration Time) eingetragen.

In Figur 5b sind die Werte für den maximalen Gradienten des Impedanzverlaufes und für die Auswurfbeschleunigungszeit für jeden der vier dargestellten Herzzyklen in ein Diagramm eingetragen. Deutlich ist zu erkennen, dass beide Herzfunktionsparameter von Herzzyklus zu Herzzyklus alternieren. Ein Blick auf Figur 5a zeigt, dass eine entsprechende Alternanz auch in Bezug auf den Maximalamplitudenwert des Impedanzverlaufes zu erkennen ist.

Es ergibt sich, dass in dem in Figur 5 dargestellten Beispiel verschiedene Herzfunktionsparameter in einem ABAB-Muster alternieren.

Die Auswerteeinheit EVAL ist ausgebildet, eine solche Alternanz für einen oder mehrere der von der Auswerteeinheit EVAL erfassten Herzfunktionsparameter zu detektieren. Zum Ermitteln einer derartigen Alternanz und/oder einer Variabilität der Herzfunktionsparameter ist die Auswerteeinheit ausgebildet, die Änderung der Herzfunktionsparameter im Zeit- oder im Frequenzbereich vorzunehmen. Eine Bestimmung der Alternanz und der Variabilität kann dabei für einen oder mehrere der Herzfunktionsparameter erfolgen.

Aus den so ermittelten Alternanzen und Variabilitäten kann die Auswerteeinheit EVAL verschiedene Risikomarkersignale ableiten. Solche Risikomarkersignale können beispielsweise als kurzfristige Prediktoren für lebensbedrohende kardiale Arrhythmien dienen, da Alternanzen oder Variabilitäten von aus der Impedanz abgeleiteten Herzfunktionsparameterwerten Prekursoren einer ventrikulären Fibrillation sein können. Die Prediktion einer ventrikulären Fibrillation geht dabei auf die Auswertung weniger jüngerer Herzzyklen zurück, stellt also eine Kurzzeit-Auswertung dar. Eine Langzeit-Auswertung der Risikoparameter kann der Vorhersage des Risikos für einen plötzlichen Herztod oder zur Beobachtung des Verlaufes einer Herzerkrankung wie beispielsweise schweren kongestiven Herzversagens dienen.

Weiterhin kann die Auswerteeinheit EVAL ausgebildet sein, eine Elektrotherapie durch den Herzschrittmacher 10 zu optimieren, das heißt insbesondere die für die elektrische Stimulation des Herzens entscheidende Parameter wie Zeitpunkt und Stärke von Stimulationsimpulsen in Abhängigkeit der von der Auswerteeinheit EVAL ermittelten Herzfunktionsparameterwerte einzustellen. Diese auf diese Weise zu optimierenden Therapieparameter schließen die Stimulationsrate und die atrioventrikuläre Verzögerungszeit ein. Bei Zweikammerschrittmachern (biventrikulären Schrittmachern) kann die Auswerteeinheit auch ausgebildet sein, auf Basis der aus der Impedanz abgeleiteten Herzfunktionsparameterwerte die interventrikuläre Verzögerungszeit zu optimieren oder den biventrikulären Stimulationsmodus. In bezug auf den biventrikulären Stimulationsmodus bestimmt die Auswerteeinheit, ob die Stimulation nur im linken Ventrikel, nur im rechten Ventrikel oder eine Stimulation beider Ventrikel erfolgen soll in Abhängigkeit davon, welche Stimulationsform zum größten Wert des Maximums des Gradienten des Impedanzverlaufes während der Systole führt. Im gleichen Sinne optimiert die Auswerteeinheit auch die interventrikuläre Verzögerungszeit.

In analoger Weise können die von der Auswerteeinheit EVAL ermittelten Herzfunktionsparameterwerte auch zur Optimierung einer Medikamententherapie ausgewertet werden.

## Patentansprüche

1. Vorrichtung zum Erfassen des Zustandes eines Herzens auf der Basis intrakardialer Impedanzmessung mit:
- einer Impedanzmesseinheit, die über elektrische Anschlüsse verfügt, mit der die Impedanzmesseinheit mit Elektroden zur Abgabe eines Stromes oder Anlegen einer Spannung sowie zum Erfassen einer Spannung bzw. eines Stromes elektrisch verbunden oder zu verbinden ist und die ausgebildet ist, eine Impedanz auf Basis der Größe des Abgegebenen Stromes oder der abgegebenen Spannung und den durch den Strom hervorgerufenen Spannungsabfall bzw. durch die Spannung hervorgerufenen Strom zu ermitteln, und mit
- einer Auswerteeinheit, die mit der Impedanzmesseinheit verbunden und ausgebildet ist, aus einem mittels der Impedanzmesseinheit ermittelten zeitlichen Verlauf der Impedanz den einer Diastole zugeordneten Verlauf des Impedanzsignals auszuwerten, und das negative Maximum des Gradienten des Impedanzverlaufes als einen das Verhalten eines Herzens während der Diastole kennzeichnenden Herzfunktionsparameter abzuleiten,
**dadurch gekennzeichnet, dass** die Auswerteeinheit ausgebildet ist, durch Auswerten des Impedanzverlaufes die Zeitdauer zwischen Beginn der Impedanzabnahme und dem darauffolgenden Auftreten des maximalen negativen Gradienten als Herzfunktionsparameter zu bestimmen.

2. Vorrichtung nach Anspruch 1, **dadurch gekennzeichnet, dass** die Auswerteeinheit ausgebildet ist, durch Auswerten des Impedanzverlaufes entweder die Zeitdauer zwischen dem Auftreten des maximalen negativen Gradienten und dem Apex einer parabolischen-Näherungsfunktion des Impedanzverlaufes beginnend mit dem Auftreten des maximalen negativen Gradienten oder der Zeitkonstante einer exponentiellen Näherungsfunktion des Impedanzverlaufes beginnend mit dem Auftreten des maximalen negativen Gradienten als Wert eines Herzfunktionsparameters, nämlich der Füllverzögerungszeit, zu bestimmen.

3. Vorrichtung nach einem der Ansprüche 1 oder2, **dadurch gekennzeichnet, dass** die Auswerteeinheit ausgebildet ist, durch Auswerten des Impedanzverlaufes das Verhältnis des negativen Maximums des Gradienten des Impedanzverlaufes zwischen dem Beginn der Abnahme der Impedanz und einer darauffolgenden atrialen Kontraktion zu dem negativen Maximum des Gradienten des Impedanzverlaufens zwischen dieser atrialen Kontraktion und dem darauffolgenden Wiederanstieg des Impedanzverlaufes als Wert eines Herzfunktionsparameters zu bestimmen.

4. Vorrichtung nach einem der Ansprüche 1 bis 3, **dadurch gekennzeichnet, dass** die Auswerteeinheit ausgebildet ist, durch Auswerten des Impedanzverlaufes die Dauer einer jeweiligen Diastole als Herzfunktionsparameterwert durch Auswerten des Impedanzverlaufes zu bestimmen, indem die Auswerteeinheit die Zeitdauer zwischen dem Beginn der Abnahme der Impedanz nach Überschreiten des Impedanzmaximums und dem Auftreten des Minimums des Impedanzverlaufes bestimmt.

5. Vorrichtung nach einem der Ansprüche 1 bis 4, **dadurch gekennzeichnet, dass** die Auswerteeinheit ausgebildet ist, den einer Systole zugeordneten Verlauf des Impedanzsignals auszuwerten und einen das Verhalten eines Herzens während der Systole (Auswurfphase der Herzkammern, Kontraktion) kennzeichnenden Herzfunktionsparameterwert zu bilden.

6. Vorrichtung nach Anspruch 5, **dadurch gekennzeichnet, dass** die Auswerteeinheit ausgebildet ist, das positive Maximum des Gradienten des Impedanzverlaufes als Herzfunktionsparameterwert zu bestimmen.

7. Vorrichtung nach Anspruch 5 oder 6, **dadurch gekennzeichnet, dass** die Auswerteeinheit ausgebildet ist, das Maximum des Produkts aus der Steigung des Gradienten des Impedanzverlaufes und dem Gradienten des Impedanzverlaufes als Herzfunktionsparameterwert zu bestimmen.

8. Vorrichtung nach einem der Ansprüche 5 bis 7, **dadurch gekennzeichnet, dass** die Auswerteeinheit ausgebildet ist, durch Auswerten des Impedanzverlaufes die Zeitdauer der Vor-Auswurf-Phase als Herzfunktionsparameterwert zu bestimmen, indem die Auswerteeinheit die Zeitdauer zwischen der elektrischen Aktivierung des ventrikulären Myocards und dem Beginn des Impedanzanstiegs bestimmt.

9. Vorrichtung nach einem der Ansprüche 5 bis 8, **dadurch gekennzeichnet, dass** die Auswerteeinheit ausgebildet ist, durch Auswerten des Impedanzverlaufes die Zeitdauer der linksventrikulären Auswurfzeit zu bestimmen, in dem die Auswerteeinheit die Zeitdauer zwischen dem Beginn des Impedanzanstiegs und dem Maximum des Impedanzverlaufes bestimmt.

10. Vorrichtung nach einem der Ansprüche 5 oder 9, **dadurch gekennzeichnet, dass** die Auswerteeinheit ausgebildet ist, durch Auswerten des Impedanzverlaufes die Zeitdauer zwischen Beginn des Impedanzanstiegs und dem darauffolgenden Auftreten des maximalen Gradienten des Impedanzverlaufes als Wert eines Herzfunktionsparameters, nämlich er der Auswurfbeschleunigungszeit, zu bestimmen.

11. Vorrichtung nach einem der Ansprüche 5 bis 10, **dadurch gekennzeichnet, dass** die Auswerteeinheit ausgebildet ist, durch Auswerten des Impedanzverlaufes die Zeitdauer zwischen dem Auftreten des maximalen Gradienten des Impedanzverlaufes und dem Maximum des Impedanzverlaufes als Wert eines Herzfunktionsparameters, nämlich der Auswurfverzögerungszeit, zu bestimmen.

12. Vorrichtung nach Anspruch 10 und 11, **dadurch gekennzeichnet, dass** die Auswerteeinheit ausgebildet ist, das Verhältnis von Auswurfbeschleunigungszeitdauer zu Auswurfverzögerungszeitdauer für einen jeweiligen Herzzyklus zu bilden.

13. Vorrichtung nach einem der Ansprüche 1 bis 12, **dadurch gekennzeichnet, dass** die Vorrichtung einen Speicher aufweist, der mit der Auswerteeinheit verbunden und dazu ausgebildet ist, von der Auswerteeinheit ermittelte Herzfunktionsparameterwerte zu speichern.

14. Vorrichtung nach Anspruch 13, **dadurch gekennzeichnet, dass** Auswerteeinheit mit einer Aktivitätserfassungseinheit verbunden ist, die ausgebildet ist, Zustände physischer Aktivität eines Patienten und Ruhezuständen des Patienten differenziert zu erfassen und ein entsprechendes Aktivitäts- bzw. Ruheausgangssignal zu erzeugen, wobei der Speicher wenigstens zwei Speicherbereiche aufweist, von denen ein erster für das Speichern von Herzfunktionsparameterwerten vorgesehen ist, die während einer Phase physischer Aktivität des Patienten erfasst wurden und ein zweiter Speicherbereich für das Speichern von Herzfunktionsparameterwerten, die während einer Ruhephase des Patienten erfasst wurden und wobei die Auswerteeinheit dazu ausgebildet ist, Herzfunktionsparameterwerte in Abhängigkeit des Ausgangssignals der Aktivitätserfassungseinheit in dem ersten oder dem zweiten Speicherbereich zu speichern.

15. Vorrichtung nach Anspruch 14, **dadurch gekennzeichnet, dass** die Aktivitätserfassungseinheit dazu ausgebildet ist, mehrere Zustände physischer Aktivität unterschiedlicher Intensität zu differenzieren, der Speicher mehrere Speicherbereiche aufweist und die Auswerteeinheit ausgebildet ist, Herzfunktionsparameterwerte in Abhängigkeit des Ausgangssignals der Aktivitätserfassungseinheit je nach Intensität der zugeordneten physischen Aktivität in einem der Speicherbereiche zu speichern.

16. Vorrichtung nach einem der Ansprüche 13 bis 15, **dadurch gekennzeichnet, dass** die Auswerteeinheit zum Bilden von Mittelwerten von Herzfunktionsparameterwerten eines Herzfunktionsparameters über einen vorgebbaren Zeitraum ausgebildet ist.

17. Vorrichtung nach einem der Ansprüche 13 bis 16, **dadurch gekennzeichnet, dass** die Auswerteeinheit ausgebildet ist, eine Alternanz eines Herzfunktionsparameters von Herzzyklus zu Herzzyklus zu erfassen.

18. Vorrichtung nach einem der Ansprüche 13 bis 17, **dadurch gekennzeichnet, dass** die Vorrichtung eine Telemetrieeinheit aufweist, die wenigstens einen Sender zum drahtlosen Übertragen von Daten umfasst und die mit dem Speicher verbunden ist.

19. Vorrichtung nach einem der Ansprüche 1 bis 18, **dadurch gekennzeichnet, dass** die Vorrichtung als Elektrostimulationsgerät ausgebildet ist.

20. Vorrichtung nach Anspruch 19, **dadurch gekennzeichnet, dass** die Vorrichtung implantierbar ist.

21. Vorrichtung nach Anspruch 18, **dadurch gekennzeichnet, dass** die Vorrichtung ein implantierbarer Herzschrittmacher oder Cardioverter/Defibrillator oder beides ist.

## Claims

1. A device for detecting the state of a heart on the basis of intracardial impedance measurement having:
- an impedance measuring unit, which has electrical terminals, using which the impedance measuring unit is electrically connected or is to be connected to electrodes for delivering a current or applying a voltage and for detecting a voltage and/or a current, and which is implemented to ascertain an impedance on the basis of the size of the delivered current or the applied voltage and the voltage drop caused by the delivered current and/or the current caused by the applied voltage, and having
- an analysis unit, which is connected to the impedance measuring unit and is implemented to analyze the time dependent curve of the impedance assigned to a diastole from a time dependent curve of the impedance ascertained using the impedance measuring unit, and to derive the negative maximum of the gradient of the impedance curve as a cardiac function parameter characterizing the behavior of a heart during the diastole,
**characterized in that** the analysis unit is implemented to determine the duration between beginning of the impedance drop and the following occurrence of the maximum negative gradient as a cardiac function parameter by analyzing the impedance curve.

2. The device according to Claim 1, **characterized in that** the analysis unit is implemented, by analyzing the impedance curve, to determine either the duration between the occurrence of the maximum negative gradient and the apex of a parabolic approximation function of the impedance curve beginning with the occurrence of the maximum negative gradient or an exponential approximation function of the impedance beginning with the occurrence of the maximum negative gradient as a value of a cardiac function parameter, namely the filling delay time.

3. The device according to one of Claims 1 or 2, **characterized in that** the analysis unit is implemented, by analyzing the impedance curve, to determine the ratio of the negative maximum of the gradient of the impedance curve between the beginning of the drop of the impedance and a following atrial contraction to the negative maximum of the gradient of the impedance curve between this atrial contraction and the following further increase of the impedance curve as a value of the cardiac function parameter.

4. The device according to one of Claims 1 through 3, **characterized in that** the analysis unit is implemented, by analyzing the impedance curve, to determine the duration of a particular diastole as a cardiac function parameter value by analyzing the impedance curve, **in that** the analysis unit determines the duration between the beginning of the drop of the impedance after exceeding the impedance maximum and the occurrence of the minimum of the impedance curve.

5. The device according to one of Claims 1 through 4, **characterized in that** the analysis unit is implemented to analyze the curve of the impedance signal assigned to a systole and to provide a cardiac function parameter value characterizing the behavior of a heart during the systole (ejection phase of the ventricle, contraction).

6. The device according to Claim 5, **characterized in that** the analysis unit is implemented to determine the positive maximum of the gradient of the impedance curve as a cardiac function parameter value.

7. The device according to Claim 5 or 6, **characterized in that** the analysis unit is implemented to determine the maximum of the product of the slope of the gradient of the impedance curve and the gradient of the impedance curve as a cardiac function parameter value.

8. The device according to one of Claims 5 through 7, **characterized in that** the analysis unit is implemented, by analyzing the impedance curve, to determine the duration of the pre-ejection phase as a cardiac function parameter value, **in that** the analysis unit determines the duration between the electrical activation of the ventricular myocardium and the beginning of the impedance rise.

9. The device according to one of Claims 5 through 8, **characterized in that** the analysis unit is implemented, by analyzing the impedance curve, to determine the duration of the left-ventricular ejection time, **in that** the analysis unit determines the duration between the beginning of the impedance rise and the maximum of the impedance curve.

10. The device according to one of Claims 5 or 9, **characterized in that** the analysis unit is implemented, by analyzing the impedance curve, to determine the duration between the beginning of the impedance rise and the following occurrence of the maximum gradient of the impedance curve as a value of a cardiac function parameter, namely the ejection acceleration time.

11. The device according to one of Claims 5 through 10, **characterized in that** the analysis unit is implemented, by analyzing the impedance curve, to determine the duration between the occurrence of the maximum gradient of the impedance curve and the maximum of the impedance curve as a value of a cardiac function parameter, namely the ejection delay time.

12. The device according to Claim 10 and 11, **characterized in that** the analysis unit is implemented to calculate the ratio of ejection acceleration duration to ejection delay duration for a particular cardiac cycle.

13. The device according to one of Claims 1 through 12, **characterized in that** the device has a memory which is connected to the analysis unit and is implemented to store cardiac function parameter values ascertained by the analysis unit.

14. The device according to Claim 13, **characterized in that** the analysis unit is connected to an activity detection unit, which is implemented to detect states of physical activity of a patient and rest state of the patient in differentiated form and to generate a corresponding activity or rest output signal, respectively, the memory having at least two storage areas, of which a first storage area is provided for storing cardiac function parameter values which were detected during a phase of physical activity and a second storage area is provided for storing cardiac function parameters which were detected during a rest phase of the patient, and the analysis unit being implemented to store cardiac function parameter values as a function of the output signal of the activity detection unit in the first or the second storage area.

15. The device according to Claim 14, **characterized in that** the activity detection unit is implemented to differentiate multiple states of physical activity of differing intensity, the memory having multiple storage areas and the analysis unit being implemented to store cardiac function parameter values in one of the storage areas as a function of the output signal of the activity detection unit depending on the intensity of the assigned physical activity.

16. The device according to one of Claims 13 through 15, **characterized in that** the analysis unit is implemented to calculate mean values of cardiac function parameter values of a cardiac function parameter over a pre-definable period of time.

17. The device according to one of Claims 13 through 16, **characterized in that** the analysis unit is implemented to detect an alternation of a cardiac function parameter from cardiac cycle to cardiac cycle.

18. The device according to one of Claims 13 through 17, **characterized in that** the device has a telemetry unit, which comprises at least one transmitter for wirelessly transmitting data and is connected to the memory.

19. The device according to one of Claims 1 through 18, **characterized in that** the device is implemented as an electrostimulation device.

20. The device according to Claim 19, **characterized in that** the device is implantable.

21. The device according to Claim 18, **characterized in that** the device is an implantable cardiac pacemaker or cardioverter/defibrillator or both.

## Revendications

1. Dispositif pour enregistrer l'état d'un coeur sur la base d'une mesure d'impédance intracardiaque comprenant :
- une unité de mesure d'impédance, qui dispose de branchements électriques, avec lesquels l'unité de mesure d'impédance est reliée ou doit peut être reliée électriquement avec des électrodes pour la délivrance d'un courant ou l'application d'une tension ainsi que pour l'enregistrement d'une tension ou d'un courant et qui est conçue pour déterminer une impédance sur la base de la grandeur du courant délivré ou de la tension délivrée et la chute de tension provoquée par le courant ou le courant provoqué par la tension, et
- une unité d'analyse qui est reliée à l'unité de mesure d'impédance et qui est conçue pour réaliser la courbe, attribuée à une diastole, du signal d'impédance à partir d'une courbe dans le temps de l'impédance, déterminée au moyen de l'unité de mesure d'impédance, et pour déduire le maximum négatif du gradient de la courbe d'impédance sous la forme d'un paramètre de fonction cardiaque caractérisant le comportement d'un coeur pendant la diastole,
**caractérisé en ce que** l'unité d'analyse est conçue pour déterminer par l'analyse de la courbe d'impédance la durée s'écoulant entre le début de la diminution d'impédance et l'apparition consécutive du gradient négatif maximum en tant que paramètre de fonction cardiaque.

2. Dispositif selon la revendication 1, **caractérisé en ce que** l'unité d'analyse est conçue pour déterminer par l'analyse de la courbe d'impédance soit la durée s'écoulant entre l'apparition du gradient négatif maximum et l'apex d'une fonction d'approximation parabolique de la courbe d'impédance commençant avec l'apparition du gradient négatif maximum ou la constante dans le temps d'une fonction d'approximation exponentielle de la courbe d'impédance en commençant avec l'apparition du gradient négatif maximum comme valeur d'un paramètre de fonction cardiaque, c'est-à-dire la temporisation de remplissage.

3. Dispositif selon l'une quelconque des revendications 1 ou 2, **caractérisé en ce que** l'unité d'analyse est conçue pour déterminer par l'analyse de la courbe d'impédance le rapport entre le maximum négatif du gradient de la courbe d'impédance entre le début de la diminution de l'impédance et une contraction atriale consécutive au maximum négatif du gradient de la courbe d'impédance entre cette contraction atriale et l'accroissement consécutif de la courbe d'impédance comme valeur d'un paramètre de fonction cardiaque.

4. Dispositif selon l'une quelconque des revendications 1 à 3, **caractérisé en ce que** l'unité d'analyse est conçue pour déterminer par l'analyse de la courbe d'impédance la durée d'une diastole respective comme valeur de paramètre de fonction cardiaque par l'analyse de la courbe d'impédance par le fait que l'unité d'analyse détermine la durée s'écoulant entre le début de la baisse de l'impédance après le dépassement du maximum d'impédance et l'apparition du minimum de la courbe d'impédance.

5. Dispositif selon l'une quelconque des revendications 1 à 4, **caractérisé en ce que** l'unité d'analyse est conçue pour analyser la courbe, attribuée à une systole, du signal d'impédance et former une valeur de paramètre de fonction cardiaque qui caractérise le comportement d'un coeur pendant la systole (phase d'éjection des ventricules, contraction).

6. Dispositif selon la revendication 5, **caractérisé en ce que** l'unité d'analyse est conçue pour déterminer le maximum positif du gradient de la courbe d'impédance comme valeur de paramètre de fonction cardiaque.

7. Dispositif selon la revendication 5 ou 6, **caractérisé en ce que** l'unité d'analyse est conçue pour déterminer le maximum du produit à partir de la pente du gradient de la courbe d'impédance et du gradient de la courbe d'impédance comme valeur de paramètre de fonction cardiaque.

8. Dispositif selon l'une quelconque des revendications 5 à 7, **caractérisé en ce que** l'unité d'analyse est conçue pour déterminer par l'analyse de la courbe d'impédance la durée de la phase de pré-éjection comme valeur de paramètre de fonction cardiaque du fait que l'unité d'analyse détermine la durée s'écoulant entre l'activation électrique du myocarde ventriculaire et le début de l'augmentation d'impédance.

9. Dispositif selon l'une quelconque des revendications 5 à 8, **caractérisé en ce que** l'unité d'analyse est conçue pour déterminer par l'analyse de la courbe d'impédance la durée de la période d'éjection du ventricule gauche du fait que l'unité d'analyse détermine la durée s'écoulant entre le début de l'augmentation d'impédance et le maximum de la courbe d'impédance.

10. Dispositif selon l'une quelconque des revendications 5 ou 9, **caractérisé en ce que** l'unité d'analyse est conçue pour déterminer par l'analyse de la courbe d'impédance la durée s'écoulant entre le début de l'augmentation d'impédance et l'apparition consécutive du gradient maximal de la courbe d'impédance comme valeur d'un paramètre de fonction cardiaque c'est-à-dire le temps d'accélération d'éjection.

11. Dispositif selon l'une quelconque des revendications 5 à 10, **caractérisé en ce que** l'unité d'analyse est conçue pour déterminer par l'analyse de la courbe d'impédance la durée s'écoulant entre l'apparition du gradient maximum de la courbe d'impédance et le maximum de la courbe d'impédance comme valeur d'un paramètre de fonction cardiaque, c'est-à-dire la temporisation d'éjection.

12. Dispositif selon les revendications 10 et 11, **caractérisé en ce que** l'unité d'analyse est conçue pour former le rapport entre la durée d'accélération d'éjection et la durée de temporisation d'éjection pour un cycle cardiaque respectif.

13. Dispositif selon l'une quelconque des revendications 1 à 12, **caractérisé en ce que** le dispositif présente une mémoire qui est reliée à l'unité d'analyse et est conçue pour stocker des valeurs de paramètre de fonction cardiaque déterminées par l'unité d'analyse.

14. Dispositif selon la revendication 13, **caractérisé en ce que** l'unité d'analyse est reliée à une unité d'enregistrement d'activité, qui est conçue pour enregistrer de façon différenciée des états d'activité physique d'un patient et des états de repos du patient et de générer un signal de départ d'activité ou de repos correspondant, la mémoire présentant au moins deux zones de mémoire, dont une première est prévue pour le stockage de valeurs de paramètre de fonction cardiaque, qui ont été enregistrées pendant une phase d'activité physique du patient, et une seconde zone de stockage pour le stockage de valeurs de paramètre de fonction cardiaque, qui ont été enregistrées pendant une phase de repos du patient et l'unité d'analyse étant conçue pour stocker des valeurs de paramètre de fonction cardiaque en fonction du signal de départ de l'unité d'enregistrement d'activité dans la première ou dans la seconde zone de mémoire.

15. Dispositif selon la revendication 14, **caractérisé en ce que** l'unité d'enregistrement d'activité est conçue pour différencier plusieurs états d'activité physique d'intensité différente, la mémoire présente plusieurs zones de mémoire et l'unité d'analyse est conçue pour stocker des valeurs de paramètre de fonction cardiaque en fonction du signal de départ de l'unité d'enregistrement d'activité en fonction de l'intensité de l'activité physique attribuée dans l'une des zones de mémoire.

16. Dispositif selon l'une quelconque des revendications 13 à 15, **caractérisé en ce que** l'unité d'analyse est conçue pour former des moyennes de valeur de paramètre de fonction cardiaque d'un paramètre de fonction cardiaque pendant une période prédéfinissable.

17. Dispositif selon l'une quelconque des revendications 13 à 16, **caractérisé en ce que** l'unité d'analyse est conçue pour enregistrer une alternance d'un paramètre de fonction cardiaque d'un cycle cardiaque à l'autre.

18. Dispositif selon l'une quelconque des revendications 13 à 17, **caractérisé en ce que** le dispositif présente une unité de télémétrie qui comporte au moins un émetteur pour la transmission sans fil de données et qui est relié à la mémoire.

19. Dispositif selon l'une quelconque des revendications 1 à 18, **caractérisé en ce que** le dispositif est conçu comme appareil d'électrostimulation.

20. Dispositif selon la revendication 19, **caractérisé en ce que** le dispositif est implantable.

21. Dispositif selon la revendication 18, **caractérisé en ce que** le dispositif est un pacemaker ou un cardiovecteur/défibrillateur implantable ou les deux.
